# EUROPEAN PATENT APPLICATION

(11) **EP 4 847 530 A1**
(43) Date of publication of application: **02.12.2026**
(21) Application number: 25744729.2
(22) Date of filing: 24.01.2025
(51) Int. Cl.: C07K 16/28, A61K 39/395, A61K 38/00, A61P 35/00, C12N 15/13

(54) **NECTIN-4 ANTIBODY AND USE THEREOF**

(30) Priority: 26.01.2024 CN 202410114460
(71) Applicant: Lunan New Time Biotechnical Co., Ltd., Linyi, Shandong 273400 (CN)
(72) Inventor: ZHAO, Lili, Linyi, Shandong 273400 (CN); LIU, Zhong, Linyi, Shandong 273400 (CN); ZHANG, Guimin, Linyi, Shandong 276006 (CN); ZHU, Zhongsong, Linyi, Shandong 273400 (CN); XU, Yongliang, Linyi, Shandong 273400 (CN); CHENG, Fanliang, Linyi, Shandong 273400 (CN); PENG, Wei, Linyi, Shandong 273400 (CN); ZHU, Yuqiang, Linyi, Shandong 273400 (CN)
(74) Representative: Inspicos P/S
(86) International application number: PCT/CN2025/074708
(87) International publication number: WO 2025/157266

(57) **Abstract**

Provided are an anti-Nectin-4 antibody or an antigen-binding fragment thereof, an antibody conjugate, and uses thereof, relating to the field of biotechnology. Specifically, the obtained Nectin-4 antibody exhibits higher affinity and favorable cellular internalization rates; the antibody conjugate comprising the antibody demonstrates significant cell-killing effects and potent tumor growth inhibitory activity. Also provided are uses of the antibody or the antigen-binding fragment thereof and the antibody conjugate in the manufacture of a medicament for the treatment of cancer, and the antibody or the antigen-binding fragment thereof and the antibody conjugate play important roles in the treatment of human tumors.

## Description

### TECHNICAL FIELD

The present invention relates to the field of biotechnology, and specifically to an antibody that specifically binds to Nectin-4 and a fragment thereof, as well as uses of such antibodies.

### BACKGROUND

The Nectin cell adhesion molecule family comprises four members: Nectin-1, Nectin-2, Nectin-3, and Nectin-4. Among these, Nectin-1, Nectin-2, and Nectin-3 are broadly expressed in normal adult tissues, whereas Nectin-4 is broadly expressed in cancerous tissues.

In recent years, Nectin cell adhesion molecule 4 (Nectin-4) has been recognized as an important factor in the formation of malignant tumors, including urothelial carcinoma, gastric cancer, thyroid cancer, breast cancer, esophageal cancer, and ovarian cancer. Nectin-4 is a member of the immunoglobulin-like adhesion molecule Nectin family. The abnormal expression of Nectin-4 is associated with tumor cell progression through proliferation, angiogenesis, and reduced apoptosis, indicating that Nectin-4 is a potential therapeutic target.

An antibody-drug conjugate (ADC) is a technology that leverages the specific recognition capability of antibodies toward particular antigens on the surface of tumor cells to precisely deliver anti-tumor drugs (such as cytotoxic agents, cytostatic agents, and small-molecule chemotherapeutics) to tumor target cells, enabling intracellular accumulation and release, thereby precisely killing tumors. An ADC generally consists of three components: an antibody or antibody-based ligand, a small-molecule drug, and a linker that conjugates the antibody or antibody-based ligand to the drug. Due to their appropriate molecular weight, high stability, strong targeting capability, and low toxicity and side effects, ADCs have been recognized as the most promising anti-tumor agents.

The primary drug currently targeting Nectin-4 is Enfortumab vedotin, an antibody-drug conjugate composed of an anti-Nectin-4 monoclonal antibody conjugated to the cytotoxic drug monomethyl auristatin E (MMAE), which is indicated primarily for bladder cancer, particularly urothelial carcinoma, and granted Breakthrough Therapy Designation by the FDA in March 2018. In addition, studies have shown that the adhesion molecule Nectin-4 can serve not only as an effective prognostic factor for breast cancer, but also as an effective therapeutic target for patients with triple-negative breast cancer (TNBC). Both in vitro and in vivo studies have confirmed that the anti-Nectin-4 antibody-drug conjugate (ADC) demonstrates favorable efficacy against locally advanced and metastatic TNBC.

Seagen (Seattle Genetics), in collaboration with Astellas, utilized its proprietary mc-vc-MMAE linker to randomly conjugate with the Anti-Nectin-4 antibody enfortumab, resulting in the generation of an Anti-Nectin-4 antibody-drug conjugate known as Enfortumab Vedotin (Padcev). Clinical trial results demonstrated that among patients receiving chemotherapy and PD-1/PD-L1 inhibitors, those receiving Enfortumab Vedotin achieved a median overall survival of 12.9 months, which was 3.9 months longer than that of the chemotherapy control group, demonstrating favorable anti-tumor efficacy. However, clinical studies have also found that the use of Enfortumab Vedotin is often associated with fever, pruritus, peripheral neuropathy, dry eye syndrome, and neutropenia. These adverse reactions are directly associated with excessive conjugation of the small molecule to the antibody and instability of the conjugation mode.

Therefore, there is an urgent need in the art to provide efficient, simple, and practical chemical conjugation methods for the research and development of antibody-drug conjugates targeting Nectin-4.

### SUMMARY

In view of the above problems, the present invention provides an antibody molecule that specifically binds to Nectin-4 or a functional fragment thereof, and an antibody conjugate comprising the antibody or the functional fragment thereof. The antibody molecule or the functional fragment thereof according to the present invention exhibits higher affinity and favorable cellular internalization rates; the antibody conjugate comprising the antibody or the functional fragment thereof demonstrates significant cell killing effects and a strong tumor inhibition rate.

In a first aspect, there is provided an anti-Nectin-4 antibody or an antigen-binding fragment thereof, comprising a heavy chain variable region and/or a light chain variable region, the heavy chain variable region comprising heavy chain CDRs selected from the amino acid sequences of SEQ ID NOs: 1-17, 46, or variants thereof; and the light chain variable region comprising light chain CDRs selected from the amino acid sequences of SEQ ID NOs: 18-30, or variants thereof.

In some embodiments, the present invention provides an anti-Nectin-4 antibody or an antigen-binding fragment thereof, comprising a heavy chain variable region and/or a light chain variable region, the heavy chain variable region comprising heavy chain complementarity-determining regions HCDR1, HCDR2, and HCDR3, and the light chain variable region comprising light chain complementarity-determining regions LCDR1, LCDR2, and LCDR3, wherein:
(1) the amino acid sequences of HCDR1, HCDR2, and HCDR3 are set forth in SEQ ID NO: 1, SEQ ID NO: 2, and SEQ ID NO: 3, respectively; and the amino acid sequences of LCDR1, LCDR2, and LCDR3 are set forth in SEQ ID NO: 18, SEQ ID NO: 19, and SEQ ID NO: 20, respectively; or (2) the amino acid sequences of HCDR1, HCDR2, and HCDR3 are set forth in SEQ ID NO: 4, SEQ ID NO: 5, and SEQ ID NO: 6, respectively; and the amino acid sequences of LCDR1, LCDR2, and LCDR3 are set forth in SEQ ID NO: 21, SEQ ID NO: 22, and SEQ ID NO: 23, respectively; or (3) the amino acid sequences of HCDR1, HCDR2, and HCDR3 are set forth in SEQ ID NO: 7, SEQ ID NO: 8, and SEQ ID NO: 9, respectively; and the amino acid sequences of LCDR1, LCDR2, and LCDR3 are set forth in SEQ ID NO: 24, SEQ ID NO: 19, and SEQ ID NO: 25, respectively; or (4) the amino acid sequences of HCDR1, HCDR2, and HCDR3 are set forth in SEQ ID NO: 7, SEQ ID NO: 10, and SEQ ID NO: 11, respectively; and the amino acid sequences of LCDR1, LCDR2, and LCDR3 are set forth in SEQ ID NO: 26, SEQ ID NO: 27, and SEQ ID NO: 25, respectively; or (5) the amino acid sequences of HCDR1, HCDR2, and HCDR3 are set forth in SEQ ID NO: 7, SEQ ID NO: 12, and SEQ ID NO: 9, respectively; and the amino acid sequences of LCDR1, LCDR2, and LCDR3 are set forth in SEQ ID NO: 24, SEQ ID NO: 19, and SEQ ID NO: 25, respectively; or (6) the amino acid sequences of HCDR1, HCDR2, and HCDR3 are set forth in SEQ ID NO: 13, SEQ ID NO: 14, and SEQ ID NO: 15, respectively; and the amino acid sequences of LCDR1, LCDR2, and LCDR3 are set forth in SEQ ID NO: 28, SEQ ID NO: 29, and SEQ ID NO: 30, respectively; or (7) the amino acid sequences of HCDR1, HCDR2, and HCDR3 are set forth in SEQ ID NO: 13, SEQ ID NO: 16, and SEQ ID NO: 17, respectively; and the amino acid sequences of LCDR1, LCDR2, and LCDR3 are set forth in SEQ ID NO: 28, SEQ ID NO: 29, and SEQ ID NO: 30, respectively; or (8) the amino acid sequences of HCDR1, HCDR2, and HCDR3 are set forth in SEQ ID NO: 13, SEQ ID NO: 46, and SEQ ID NO: 17, respectively; and the amino acid sequences of LCDR1, LCDR2, and LCDR3 are set forth in SEQ ID NO: 28, SEQ ID NO: 29, and SEQ ID NO: 30, respectively.

In some preferred embodiments, for the antibody or the antigen-binding fragment thereof, the amino acid sequences of HCDR1, HCDR2, HCDR3 of the heavy chain variable region and the amino acid sequences of LCDR1, LCDR2, and LCDR3 of the light chain variable region are selected from the group consisting of:
(1) the amino acid sequences of HCDR1, HCDR2, and HCDR3 are set forth in SEQ ID NO: 13, SEQ ID NO: 16, and SEQ ID NO: 17, respectively; and the amino acid sequences of LCDR1, LCDR2, and LCDR3 are set forth in SEQ ID NO: 28, SEQ ID NO: 29, and SEQ ID NO: 30, respectively; or (2) the amino acid sequences of HCDR1, HCDR2, and HCDR3 are set forth in SEQ ID NO: 13, SEQ ID NO: 46, and SEQ ID NO: 17, respectively; and the amino acid sequences of LCDR1, LCDR2, and LCDR3 are set forth in SEQ ID NO: 28, SEQ ID NO: 29, and SEQ ID NO: 30, respectively.

In a second aspect of the present invention, there is provided an anti-Nectin-4 antibody or an antigen-binding fragment thereof, wherein the antibody or the antigen-binding fragment thereof comprises a heavy chain variable region (VH) and a light chain variable region (VL): an amino acid sequence of the heavy chain variable region comprises an amino acid sequence selected from the group consisting of SEQ ID NOs: 31-37 and SEQ ID NOs: 47-52, and an amino acid sequence of the light chain variable region comprises an amino acid sequence selected from the group consisting of SEQ ID NOs: 38-43 and SEQ ID NOs: 53-57; or an amino acid sequence having at least 80%, 85%, 90%, 95%, 96%, 97%, 98%, or 99% identity to the amino acid sequence selected from the group consisting of SEQ ID NOs: 31-37, SEQ ID NOs: 47-52, SEQ ID NOs: 38-43, and SEQ ID NOs: 53-57.

In some embodiments, the antibody of the invention comprises a combination of a heavy chain variable region (VH) and a light chain variable region (VL) selected from the following possibilities:
(1) the amino acid sequence of the heavy chain variable region set forth in SEQ ID NO: 31, and the amino acid sequence of the light chain variable region set forth in SEQ ID NO: 38; or an amino acid sequence having at least 80%, 85%, 90%, 95%, 96%, 97%, 98%, or 99% identity to the amino acid sequences; or (2) the amino acid sequence of the heavy chain variable region set forth in SEQ ID NO: 32, and the amino acid sequence of the light chain variable region set forth in SEQ ID NO: 39; or an amino acid sequence having at least 80%, 85%, 90%, 95%, 96%, 97%, 98%, or 99% identity to the amino acid sequences; or (3) the amino acid sequence of the heavy chain variable region set forth in SEQ ID NO: 33, and the amino acid sequence of the light chain variable region set forth in SEQ ID NO: 40; or an amino acid sequence having at least 80%, 85%, 90%, 95%, 96%, 97%, 98%, or 99% identity to the amino acid sequences; or (4) the amino acid sequence of the heavy chain variable region set forth in SEQ ID NO: 34, and the amino acid sequence of the light chain variable region set forth in SEQ ID NO: 41; or an amino acid sequence having at least 80%, 85%, 90%, 95%, 96%, 97%, 98%, or 99% identity to the amino acid sequences; or (5) the amino acid sequence of the heavy chain variable region set forth in SEQ ID NO: 35, and the amino acid sequence of the light chain variable region set forth in SEQ ID NO: 40; or an amino acid sequence having at least 80%, 85%, 90%, 95%, 96%, 97%, 98%, or 99% identity to the amino acid sequences; or (6) the amino acid sequence of the heavy chain variable region set forth in SEQ ID NO: 36, and the amino acid sequence of the light chain variable region set forth in SEQ ID NO: 42; or an amino acid sequence having at least 80%, 85%, 90%, 95%, 96%, 97%, 98%, or 99% identity to the amino acid sequences; or (7) the amino acid sequence of the heavy chain variable region set forth in SEQ ID NO: 37, and the amino acid sequence of the light chain variable region set forth in SEQ ID NO: 43; or an amino acid sequence having at least 80%, 85%, 90%, 95%, 96%, 97%, 98%, or 99% identity to the amino acid sequences; or (8) the amino acid sequence of the heavy chain variable region set forth in SEQ ID NO: 47, and the amino acid sequence of the light chain variable region set forth in SEQ ID NO: 43; or an amino acid sequence having at least 80%, 85%, 90%, 95%, 96%, 97%, 98%, or 99% identity to the amino acid sequences; or (9) the amino acid sequence of the heavy chain variable region set forth in SEQ ID NO: 48, and the amino acid sequence of the light chain variable region set forth in SEQ ID NO: 53; or an amino acid sequence having at least 80%, 85%, 90%, 95%, 96%, 97%, 98%, or 99% identity to the amino acid sequences; or (10) the amino acid sequence of the heavy chain variable region set forth in SEQ ID NO: 48, and the amino acid sequence of the light chain variable region set forth in SEQ ID NO: 54; or an amino acid sequence having at least 80%, 85%, 90%, 95%, 96%, 97%, 98%, or 99% identity to the amino acid sequences; or (11) the amino acid sequence of the heavy chain variable region set forth in SEQ ID NO: 48, and the amino acid sequence of the light chain variable region set forth in SEQ ID NO: 55; or an amino acid sequence having at least 80%, 85%, 90%, 95%, 96%, 97%, 98%, or 99% identity to the amino acid sequences; or (12) the amino acid sequence of the heavy chain variable region set forth in SEQ ID NO: 48, and the amino acid sequence of the light chain variable region set forth in SEQ ID NO: 56; or an amino acid sequence having at least 80%, 85%, 90%, 95%, 96%, 97%, 98%, or 99% identity to the amino acid sequences; or (13) the amino acid sequence of the heavy chain variable region set forth in SEQ ID NO: 48, and the amino acid sequence of the light chain variable region set forth in SEQ ID NO: 57; or an amino acid sequence having at least 80%, 85%, 90%, 95%, 96%, 97%, 98%, or 99% identity to the amino acid sequences; or (14) the amino acid sequence of the heavy chain variable region set forth in SEQ ID NO: 49, and the amino acid sequence of the light chain variable region set forth in SEQ ID NO: 53; or an amino acid sequence having at least 80%, 85%, 90%, 95%, 96%, 97%, 98%, or 99% identity to the amino acid sequences; or (15) the amino acid sequence of the heavy chain variable region set forth in SEQ ID NO: 49, and the amino acid sequence of the light chain variable region set forth in SEQ ID NO: 54; or an amino acid sequence having at least 80%, 85%, 90%, 95%, 96%, 97%, 98%, or 99% identity to the amino acid sequences; or (16) the amino acid sequence of the heavy chain variable region set forth in SEQ ID NO: 49, and the amino acid sequence of the light chain variable region set forth in SEQ ID NO: 55; or an amino acid sequence having at least 80%, 85%, 90%, 95%, 96%, 97%, 98%, or 99% identity to the amino acid sequences; or (17) the amino acid sequence of the heavy chain variable region set forth in SEQ ID NO: 49, and the amino acid sequence of the light chain variable region set forth in SEQ ID NO: 56; or an amino acid sequence having at least 80%, 85%, 90%, 95%, 96%, 97%, 98%, or 99% identity to the amino acid sequences; or (18) the amino acid sequence of the heavy chain variable region set forth in SEQ ID NO: 49, and the amino acid sequence of the light chain variable region set forth in SEQ ID NO: 57; or an amino acid sequence having at least 80%, 85%, 90%, 95%, 96%, 97%, 98%, or 99% identity to the amino acid sequences; or (19) the amino acid sequence of the heavy chain variable region set forth in SEQ ID NO: 50, and the amino acid sequence of the light chain variable region set forth in SEQ ID NO: 53; or an amino acid sequence having at least 80%, 85%, 90%, 95%, 96%, 97%, 98%, or 99% identity to the amino acid sequences; or (20) the amino acid sequence of the heavy chain variable region set forth in SEQ ID NO: 50, and the amino acid sequence of the light chain variable region set forth in SEQ ID NO: 54; or an amino acid sequence having at least 80%, 85%, 90%, 95%, 96%, 97%, 98%, or 99% identity to the amino acid sequences; or (21) the amino acid sequence of the heavy chain variable region set forth in SEQ ID NO: 50, and the amino acid sequence of the light chain variable region set forth in SEQ ID NO: 55; or an amino acid sequence having at least 80%, 85%, 90%, 95%, 96%, 97%, 98%, or 99% identity to the amino acid sequences; or (22) the amino acid sequence of the heavy chain variable region set forth in SEQ ID NO: 50, and the amino acid sequence of the light chain variable region set forth in SEQ ID NO: 56; or an amino acid sequence having at least 80%, 85%, 90%, 95%, 96%, 97%, 98%, or 99% identity to the amino acid sequences; or (23) the amino acid sequence of the heavy chain variable region set forth in SEQ ID NO: 50, and the amino acid sequence of the light chain variable region set forth in SEQ ID NO: 57; or an amino acid sequence having at least 80%, 85%, 90%, 95%, 96%, 97%, 98%, or 99% identity to the amino acid sequences; or (24) the amino acid sequence of the heavy chain variable region set forth in SEQ ID NO: 51, and the amino acid sequence of the light chain variable region set forth in SEQ ID NO: 53; or an amino acid sequence having at least 80%, 85%, 90%, 95%, 96%, 97%, 98%, or 99% identity to the amino acid sequences; or (25) the amino acid sequence of the heavy chain variable region set forth in SEQ ID NO: 51, and the amino acid sequence of the light chain variable region set forth in SEQ ID NO: 54; or an amino acid sequence having at least 80%, 85%, 90%, 95%, 96%, 97%, 98%, or 99% identity to the amino acid sequences; or (26) the amino acid sequence of the heavy chain variable region set forth in SEQ ID NO: 51, and the amino acid sequence of the light chain variable region set forth in SEQ ID NO: 55; or an amino acid sequence having at least 80%, 85%, 90%, 95%, 96%, 97%, 98%, or 99% identity to the amino acid sequences; or (27) the amino acid sequence of the heavy chain variable region set forth in SEQ ID NO: 51, and the amino acid sequence of the light chain variable region set forth in SEQ ID NO: 56; or an amino acid sequence having at least 80%, 85%, 90%, 95%, 96%, 97%, 98%, or 99% identity to the amino acid sequences; or (28) the amino acid sequence of the heavy chain variable region set forth in SEQ ID NO: 51, and the amino acid sequence of the light chain variable region set forth in SEQ ID NO: 57; or an amino acid sequence having at least 80%, 85%, 90%, 95%, 96%, 97%, 98%, or 99% identity to the amino acid sequences; or (29) the amino acid sequence of the heavy chain variable region set forth in SEQ ID NO: 52, and the amino acid sequence of the light chain variable region set forth in SEQ ID NO: 53; or an amino acid sequence having at least 80%, 85%, 90%, 95%, 96%, 97%, 98%, or 99% identity to the amino acid sequences; or (30) the amino acid sequence of the heavy chain variable region set forth in SEQ ID NO: 52, and the amino acid sequence of the light chain variable region set forth in SEQ ID NO: 54; or an amino acid sequence having at least 80%, 85%, 90%, 95%, 96%, 97%, 98%, or 99% identity to the amino acid sequences; or (31) the amino acid sequence of the heavy chain variable region set forth in SEQ ID NO: 52, and the amino acid sequence of the light chain variable region set forth in SEQ ID NO: 55; or an amino acid sequence having at least 80%, 85%, 90%, 95%, 96%, 97%, 98%, or 99% identity to the amino acid sequences; or (32) the amino acid sequence of the heavy chain variable region set forth in SEQ ID NO: 52, and the amino acid sequence of the light chain variable region set forth in SEQ ID NO: 56; or an amino acid sequence having at least 80%, 85%, 90%, 95%, 96%, 97%, 98%, or 99% identity to the amino acid sequences; or (33) the amino acid sequence of the heavy chain variable region set forth in SEQ ID NO: 52, and the amino acid sequence of the light chain variable region set forth in SEQ ID NO: 57; or an amino acid sequence having at least 80%, 85%, 90%, 95%, 96%, 97%, 98%, or 99% identity to the amino acid sequences.

In some preferred embodiments, the antibody of the present invention is selected from the group consisting of: (1) the amino acid sequence of the heavy chain variable region set forth in SEQ ID NO: 37, and the amino acid sequence of the light chain variable region set forth in SEQ ID NO: 43; or (2) the amino acid sequence of the heavy chain variable region set forth in SEQ ID NO: 47, and the amino acid sequence of the light chain variable region set forth in SEQ ID NO: 43; or (3) the amino acid sequence of the heavy chain variable region set forth in SEQ ID NO: 48, and the amino acid sequence of the light chain variable region set forth in SEQ ID NO: 55; or (4) the amino acid sequence of the heavy chain variable region set forth in SEQ ID NO: 48, and the amino acid sequence of the light chain variable region set forth in SEQ ID NO: 57; or (5) the amino acid sequence of the heavy chain variable region set forth in SEQ ID NO: 49, and the amino acid sequence of the light chain variable region set forth in SEQ ID NO: 55; or (6) the amino acid sequence of the heavy chain variable region set forth in SEQ ID NO: 49, and the amino acid sequence of the light chain variable region set forth in SEQ ID NO: 56.

In a third aspect, there is provided an anti-Nectin-4 antibody or an antigen-binding fragment thereof, wherein the antibody or the antigen-binding fragment thereof is murine, chimeric, or humanized; wherein the framework region (FR) sequences on the light chain variable regions and the heavy chain variable regions of the humanized antibody are respectively derived from human germline light chain sequences and heavy chain sequences or mutated sequences thereof; the antibody of the present invention further comprises a heavy chain constant region and a light chain constant region.

Preferably, the antibody comprises a heavy chain constant domain of human IgG1, IgG2, IgG3, or IgG4, and a light chain constant domain of human κ or λ type, or a variant thereof, wherein the variant comprises a substitution of one or several amino acids.

Preferably, the antibody comprises a human IgG1 constant domain or a variant thereof, and a κ constant domain or a variant thereof, wherein the variant comprises a substitution of one or several amino acids.

More preferably, the antibody comprises a heavy chain constant region set forth in SEQ ID NO: 44 and a light chain constant region set forth in SEQ ID NO: 45.

More preferably, the antibody of the present invention comprises a complete structure having two light chains and two heavy chains, and the antibody is selected from the group consisting of: (1) a heavy chain amino acid sequence set forth in SEQ ID NO: 58, and a light chain amino acid sequence set forth in SEQ ID NO: 62; or (2) a heavy chain amino acid sequence set forth in SEQ ID NO: 59, and a light chain amino acid sequence set forth in SEQ ID NO: 63; or (3) a heavy chain amino acid sequence set forth in SEQ ID NO: 60, and a light chain amino acid sequence set forth in SEQ ID NO: 64; or (4) a heavy chain amino acid sequence set forth in SEQ ID NO: 61, and a light chain amino acid sequence set forth in SEQ ID NO: 65.

According to a fourth aspect of the present invention, there is provided an isolated polynucleotide molecule encoding the antibody or the antigen-binding fragment thereof according to the present invention.

In some embodiments, the polynucleotide molecule has a heavy chain nucleotide sequence set forth in SEQ ID NOs: 66-69, and a light chain nucleotide sequence set forth in SEQ ID NOs: 70-73.

In some preferred embodiments, the nucleotide molecule encoding the antibody or the antigen-binding fragment thereof according to the present invention is selected from the group consisting of: (1) the polynucleotide molecule having a heavy chain variable region nucleotide sequence set forth in SEQ ID NO: 66, and a light chain variable region nucleotide sequence set forth in SEQ ID NO: 70; or (2) the polynucleotide molecule having a heavy chain variable region nucleotide sequence set forth in SEQ ID NO: 67, and a light chain variable region nucleotide sequence set forth in SEQ ID NO: 71; or (3) the polynucleotide molecule having a heavy chain variable region nucleotide sequence set forth in SEQ ID NO: 68, and a light chain variable region nucleotide sequence set forth in SEQ ID NO: 72; or (4) the polynucleotide molecule having a heavy chain variable region nucleotide sequence set forth in SEQ ID NO: 69, and a light chain variable region nucleotide sequence set forth in SEQ ID NO: 73.

In a fifth aspect, there is provided an expression vector, wherein the expression vector comprises an isolated polynucleotide encoding the monoclonal antibody or the antigen-binding fragment thereof of the present invention.

In a sixth aspect, there is provided a host cell, wherein the host cell comprises the expression vector described above. The host cell may be a prokaryotic cell or a eukaryotic cell. In a preferred embodiment, the host cell is an Expi 293F cell.

In a seventh aspect, there is provided an antibody conjugate, comprising the anti-Nectin-4 antibody or the antigen-binding fragment thereof as described above and an effector molecule. Among them, the effector molecule is conjugated to the anti-Nectin-4 antibody.

In some embodiments, the effector molecule is selected from a radioisotope, an antineoplastic agent, an immunomodulator, a biological response modifier, a lectin, a cytotoxic drug, a chromophore, a fluorophore, a chemiluminescent compound, an enzyme, a metal ion, and any combination thereof.

In some embodiments, the antibody conjugate of the present invention has a structure as shown below: 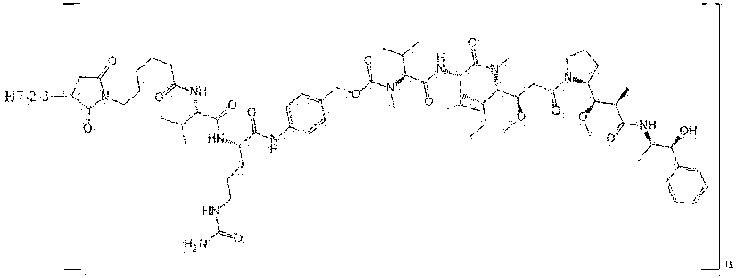
wherein n is 1 to 8;
H7-2-3 is an anti-Nectin-4 antibody, wherein the amino acid sequences of HCDR1, HCDR2, and HCDR3 of the antibody are set forth in SEQ ID NO: 13, SEQ ID NO: 46, and SEQ ID NO: 17, respectively, and the amino acid sequences of LCDR1, LCDR2, and LCDR3 of the antibody are set forth in SEQ ID NO: 28, SEQ ID NO: 29, and SEQ ID NO: 30, respectively.

Further, the antibody conjugate of the present invention has a structure as shown below: 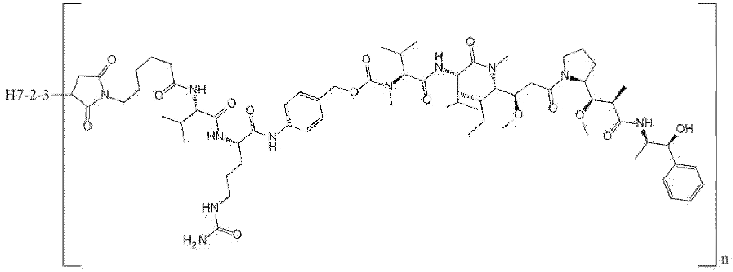
wherein n is 1 to 8;
H7-2-3 is an anti-Nectin-4 antibody, wherein the antibody has a heavy chain variable region amino acid sequence set forth in SEQ ID NO: 49, and a light chain variable region amino acid sequence set forth in SEQ ID NO: 55.

In an eighth aspect, there is provided a method for preparing the antibody that specifically binds to Nectin-4 or the antigen-binding fragment thereof according to the first aspect. The antibodies of the present invention may be produced by a variety of techniques, including conventional monoclonal antibody methods, for example, the standard somatic cell hybridization technique of Kohler and Milstein, Nature 256: 495 (1975). Somatic cell hybridization is preferred; in principle, other techniques for producing monoclonal antibodies may also be employed, for example, viral or oncogenic transformation of B lymphocytes or phage display technology using antibody gene libraries, all of which can be used to prepare the antibodies described in the present invention. Chimeric or humanized antibodies are also well known in the art, see, e.g., U.S. Patent Nos. 4,816,567, 5,225,539, 5,530,101, 5,585,089, 5,693,762, or 6,180,370.

A preferred animal system for producing hybridomas that secrete monoclonal antibodies is the murine system. Hybridoma production in mice is a well-established procedure. Immunization protocols and techniques for isolating immunized splenocytes for fusion are known in the art. Fusion partners (e.g., murine myeloma cells) and fusion methods are also known.

Other preferred animal systems for producing hybridomas that secrete monoclonal antibodies are the rat and rabbit systems (described, e.g., in Spieker-Polet et al., Proc. Natl. Acad. Sci. U.S.A. 92: 9348 (1995); see also Rossi et al., Am. J. Clin. Pathol. 124: 295 (2005)).

Another strategy for generating monoclonal antibodies involves the direct isolation of antibody-encoding genes from lymphocytes that produce antibodies of a defined strategy, see, e.g., Babcock et al., 1996; A novel strategy for generating monoclonal antibodies from single, isolated lymphocytes producing antibodies of defined specificities. For further details on recombinant antibody engineering, see also Welschof and Kraus, Recombinant antibodies for cancer therapy ISBN-0-89603-918-8and Benny K.C.Lo Antibody Engineering ISBN 1-58829-092-1.

In a ninth aspect, there are provided uses of the antibody or the antigen-binding fragment thereof and the antibody conjugate in the manufacture of a medicament for the treatment of cancer. The cancer includes, but is not limited to, bladder cancer, breast cancer, ovarian cancer, pancreatic cancer, hepatocellular carcinoma, gastric cancer, non-Hodgkin's lymphoma, Hodgkin's lymphoma, acute lymphoblastic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, a gastrointestinal tumor, prostate cancer, colorectal cancer, glioma, and mesothelioma.

The technical solution of the present invention achieves beneficial technical effects: the Nectin-4 antibody obtained in the present application exhibits higher affinity and favorable cellular internalization rates; the antibody conjugate comprising the antibody demonstrates significant cell-killing effects and a strong tumor inhibition rate.

### BRIEF DESCRIPTION OF THE DRAWINGS

FIG. 1: Results of the binding ability assay of the chimeric antibody to Nectin-4 protein;
FIG. 2: Results of the competitive binding assay of the chimeric antibody with Nectin-1 to Nectin-4;
FIG. 3: Determination results of the binding ability of the humanized antibody to Nectin-4;
FIG. 4: Results of the competitive binding assay of the humanized antibody with Nectin-1 to Nectin-4;
FIG. 5: Determination results of the binding ability of the humanized antibody to FACS (Fluorescence-Activated Cell Sorting);
FIG. 6: Results of the cytotoxic activity of the antibody-drug conjugate against tumor cells in vitro;
FIG. 7: Changes in tumor volume in a subcutaneous xenograft model of human pancreatic cancer BxPC-3 in female NOG mice treated with the conjugated antibody;
FIG. 8: Changes in body weight in a subcutaneous xenograft model of human pancreatic cancer BxPC-3 in female NOG mice treated with the conjugated antibody.

### DETAILED DESCRIPTION OF EMBODIMENTS

The present invention is further explained and illustrated below with reference to the examples. It should be understood that the following examples are merely for illustrating the present invention and are not intended to limit the scope of protection of the present invention.

In the following examples, all materials used in the experiments are commercially available or can be prepared according to existing published techniques; those without indicated sources and specifications are commercially available; various processes and methods not described in detail are conventional methods well known in the art.

The positive control antibody used in the examples was Enfortumab, prepared according to the amino acid sequences in patent CN103402538A (the heavy chain amino acid sequence set forth in SEQ ID NO: 74, the light chain amino acid sequence set forth in SEQ ID NO: 75). The sequence of human Nectin-4 protein (Q96NY8) was obtained from UniProt, with the amino acid sequence set forth in SEQ ID NO: 76, wherein amino acids 32 to 349 constitute the extracellular domain. A signal peptide and Kozak sequence were added to the N-terminus of the protein sequence, and a 6×His tag was added to the C-terminus. After sequence optimization by Nanjing GenScript Biotech Co., Ltd., it was cloned into the pcDNA3.4 vector, transiently transfected into 293F cells, and purified to obtain the Nectin-4-His protein, the amino acid sequence of which is set forth in SEQ ID NO: 77.

### Example 1 Screening and Identification of Hybridoma Cells

Five female BALB/c mice aged 6-8 weeks were selected for immunization. For the primary immunization, the antigen protein Nectin-4-His was mixed with complete Freund's adjuvant at a volume ratio of 1:1 and thoroughly emulsified using a single-channel emulsifier, followed by primary immunization of mice via heel and subcutaneous injection at a dose of 50 µg per mouse (100 µL per mouse). The second immunization was performed 14 days after the primary immunization, wherein Nectin-4-His antigen protein was mixed with incomplete Freund's adjuvant at a volume ratio of 1:1 and thoroughly emulsified using a single-channel emulsifier, followed by subcutaneous injection into mice for immunization at a dose of 50 µg per mouse. The third immunization was carried out in the same manner as the second one. Seven days after the third immunization, blood was collected from the tail vein, and antibody titers were measured by enzyme-linked immunosorbent assay (ELISA).

Three days before cell fusion, 150 µg of Nectin-4-His antigen protein was directly injected intraperitoneally into the mice for booster immunization. Three days later, the mice were euthanized, and the spleens were removed and fused with the mouse myeloma SP2/0 cell line. The prepared SP2/0 cells were mixed with mouse splenocytes at a ratio of 1:5 to 1:10, and the cell density was adjusted to 1-2×10⁷ cells/mL with BTX Buffer. Fusion was performed using an electrofusion apparatus. After fusion, HAT medium was added, and the cells were plated at 3×10 ⁴ cells per well and cultured in a 37°C, 5% CO₂ incubator.

After 14 days of culture, positive hybridoma cells were detected by ELISA. Nectin-4-His protein was coated onto 96-well ELISA plates at a concentration of 5 µg/mL (100 µL/well) and coated overnight at 4°C. The next day, the coating solution was discarded, and 200 µL/well of 5% non-fat milk was added. The plates were blocked at 37°C for 2 h, the plates were washed three times with PBST, and 50 µL of hybridoma cell culture supernatant was added, followed by incubation at 37°C for 1 h. After washing three times with PBST, the secondary antibody (goat anti-mouse IgG-HRP) was added and incubated at 37°C for 1 h. The plates were washed three times with PBST, and a chromogenic substrate solution was added for color development for 10 min, and the color reaction was terminated.

Flow cytometry was used to detect the binding ability of hybridoma cell culture supernatants to Nectin-4 expressed in 293F cells. 293F-Nectin-4 cells were collected and plated at 10 ⁵ cells/well in 96-well plates and washed once with PBS. 100 µL of hybridoma cell culture supernatant was added to the cells and incubated at 37°C for 1 h. After incubation, the plates were centrifuged once at 1000 r/min, and the supernatant was gently aspirated. The plates were washed twice with PBS at the same speed, and 100 µL of fluorescent secondary antibody in DPBS was added to each well, followed by reaction at 37°C for 1 h. After completion of the reaction, the plates were centrifuged and washed twice with PBS, and finally 200 µL of PBS was added to each well to resuspend the cells for detection by flow cytometry. The obtained positive clones were subjected to single-cell cloning, and seven hybridoma cell lines with optimal binding activity were finally screened, namely 8A6-A9, 20H6-G5, 38A3-E7, 48F6-C3, 50D3-C10, 56H10-B1, and 75B10-A11.

### Example 2 Obtaining Monoclonal Antibody Sequences

The seven screened hybridoma cell lines were cultured, RNA was extracted from each, and cDNA was amplified by RT-PCR. Hybridoma cell culture supernatants were collected, and the antibody isotypes were determined using the IsoStrip^{TM} Mouse Monoclonal Antibody Isotyping Kit. Based on the isotyping results, specific nested PCR primers were designed, and RACE PCR (GenScript) was used to amplify the heavy chain and light chain variable regions of the seven antibodies. The PCR products were subcloned into the pMD18-T vector system (TaKaRa). Insert fragments were verified and sequenced using vector-specific primers. Amino acid/DNA sequences encoding the heavy chain variable regions and light chain variable regions of the generated antibodies were obtained. The heavy chain CDR sequences, light chain CDR sequences, and the heavy chain variable region and light chain variable region amino acid sequences of the antibodies are shown in SEQ ID NOs: 1-43 and are listed in Table 1. The CDRs are determined according to the Kabat numbering system.

**Table 1 Sequence listing of heavy chain and light chain variable regions of anti-Nectin-4 hybridoma antibodies**

| Antibody | HCDR1 | HCDR2 | HCDR3 | LCDR1 | LCDR2 | LCDR3 | VH | VL |
|---|---|---|---|---|---|---|---|---|
| 8A6-A9 | 1 | 2 | 3 | 18 | 19 | 20 | 31 | 38 |
| 20H6-G5 | 4 | 5 | 6 | 21 | 22 | 23 | 32 | 39 |
| 38A3-E7 | 7 | 8 | 9 | 24 | 19 | 25 | 33 | 40 |
| 48F6-C3 | 7 | 10 | 11 | 26 | 27 | 25 | 34 | 41 |
| 50D3-C10 | 7 | 12 | 9 | 24 | 19 | 25 | 35 | 40 |
| 56H10-B1 | 13 | 14 | 15 | 28 | 29 | 30 | 36 | 42 |
| 75B10-A11 | 13 | 16 | 17 | 28 | 29 | 30 | 37 | 43 |

### Example 3 Preparation of Chimeric Antibodies

The obtained murine heavy chain variable region sequences were linked to the heavy chain constant region of human IgG1 antibody, and the light chain variable region sequences were linked to the κ light chain constant region (the heavy chain constant region amino acid sequence is set forth in SEQ ID NO: 44, and the light chain constant region amino acid sequence is set forth in SEQ ID NO: 45). A signal peptide sequence and a Kozak sequence were added upstream of the antibody amino acid sequences. DNA sequence optimization and synthesis were performed by GenScript Biotech Co., Ltd. After sequence synthesis, they were cloned into the pCDNA3.4 vector (Invitrogen). Plasmids were extracted and transfected into 293F cells. The 293F cells were cultured to the logarithmic growth phase, and the cell density was adjusted to 3×10⁶ cells/mL with Expi 293F expression medium. In 6 mL of OPTI MEM medium, 40 µg each of light chain plasmid and heavy chain plasmid were added; in another 6 mL of OPTI MEM medium, 320 µL of Expi Fectamine^{TM} 293 transfection reagent was added. The transfection reagent was added to the DNA mixture, mixed thoroughly, and allowed to stand for 15 min, then added to the cells. The cells were cultured at 37°C, 8% CO₂, 100 r/min for 20 h, after which 600 µL of Enhancer I and 6 mL of Enhancer II were added. After 5 days of culture, the samples were harvested and purified. The obtained chimeric antibodies were designated as chi-1, chi-2, chi-3, chi-4, chi-5, chi-6, and chi-7, respectively.

### Example 4 Purification and Identification of Chimeric Antibodies

First, a Protein A affinity column was prepared and equilibrated with PBS (pH 7.4). The cell culture supernatant, after centrifugation (1500 r/min, 10 min) and filtration through a 0.45 µm filter membrane, was loaded onto the column. The column was then washed with PBS (pH 7.4) until the OD₄₅₀ value approached zero. Elution was performed with 50 mmol/L glycine-hydrochloric acid buffer (pH 3.5) (50 mmol/L glycine solution adjusted to pH 3.5 with 1 mol/L hydrochloric acid). The eluate from the peak fraction was collected, adjusted to pH 6.0, and the purified antibody was obtained and stored at -20°C for later use.

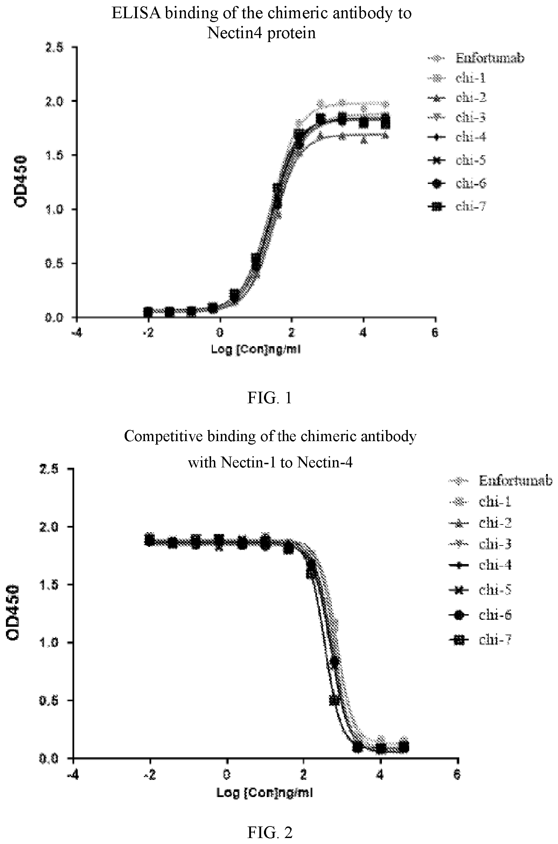

Nectin-4-His protein was diluted to 0.5 µg/mL and coated onto 96-well plates at 100 µL per well, followed by overnight coating at 4°C. The next day, the supernatant was discarded, and 300 µL of 2% non-fat milk was added to block at 37°C for 2 h. After removal of the blocking solution, the plates were washed three times with PBST. The chimeric antibodies were diluted to 40 µg/mL, and subjected to 4-fold dilution to prepare 12 gradients, then coated onto 96-well plates at 100 µL per well, followed by reaction at 37°C for 1 h. After completion of the reaction, the plates were washed three times with PBST, and 100 µL of goat anti-human IgG-Fc secondary antibody was added, followed by reaction at 37°C for 1 h. The plates were washed three times with PBST again, then 100 µL of TMB chromogenic substrate solution was added for color development for 10 min. After completion of the reaction, 100 µL1 M hydrochloric acid solution was added to each well to terminate the reaction, and the data were read and recorded at 450 nm. The results are shown in FIG. 1 and Table 2. The chimeric antibodies and the control antibody exhibited comparable binding ability for Nectin-4 protein.

**Table 2 EC₅₀ values of chimeric antibodies binding to Nectin-4**

| Antibody | EC₅₀ value (ng/mL) |
|---|---|
| chi-1 | 38.85 |
| chi-2 | 30.47 |
| chi-3 | 31.38 |
| chi-4 | 26.89 |
| chi-5 | 26.17 |
| chi-6 | 30.82 |
| chi-7 | 22.27 |
| Enfortumab | 26.97 |

### Example 5 Competitive ELISA for Identifying the Competitive Ability of Chimeric Antibodies with Nectin-4 Ligand Nectin1

Nectin-4-His protein was diluted to 2 µg/mL and coated onto 96-well plates at 100 µL per well, followed by overnight coating at 4°C. The next day, the supernatant was discarded, and 300 µL of 2% non-fat milk was added to block at 37°C for 2 h. After removal of the blocking solution, the plates were washed three times with PBST. The chimeric antibodies were diluted to 40 µg/mL and subjected to 4-fold dilution to prepare 12 gradients. The diluted antibodies were mixed with Nectin1 solution (2 µg/mL) at a 1:1 ratio (50 µL each), coated onto 96-well plates, followed by reaction at 37°C for 1 h. After completion of the reaction, the plates were washed three times with PBST, and 100 µL of goat anti-human IgG-Fc secondary antibody was added, followed by reaction at 37°C for 1 h. The plates were washed three times with PBST again, then 100 µL of TMB chromogenic substrate solution was added for color development for 10 min. After completion of the reaction, 100 µL1 M hydrochloric acid solution was added to each well to terminate the reaction, and the data were read and recorded at 450 nm. The results are shown in FIG. 2 and Table 3. The chimeric antibody chi-7 exhibited superior competitive binding ability to Nectin-4 compared to the control antibody.

**Table 3 EC₅₀ Values of chimeric antibodies competing with Nectin1 for binding to Nectin-4**

| Antibody | EC₅₀ value (ng/mL) |
|---|---|
| chi-1 | 759.7 |
| chi-2 | 550.4 |
| chi-3 | 622.6 |
| chi-4 | 537.2 |
| chi-5 | 500.4 |
| chi-6 | 532.1 |
| chi-7 | 352.1 |
| Enfortumab | 696.1 |

### Example 6 Affinity Determination of Chimeric Antibodies

The affinity of monoclonal antibodies to the antigen Nectin-4 was determined using Biacore (T200). Ultrapure water was filtered through a 0.22 µm filter membrane, and HBS-EP buffer was prepared. The Nectin-4 monoclonal antibody stock solution was diluted to 6 µg/mL with HBS-EP buffer as the ligand. Nectin-4 protein was diluted 2-fold to prepare 8 gradients starting from 100 nM using HBS-EP buffer, and a zero-concentration sample was additionally prepared as the analyte. An appropriate amount of Glycine 1.5 was taken as the regeneration solution. The ligand, analyte, regeneration solution, and HBS-EP buffer were placed on the sample tray. The program was set as follows: ligand flow rate 10 µL/min for 20 s; analyte 30 µL/min, association time 100 s, dissociation time 600 s; regeneration solution 30 µL/min for 30 s. The program was started. SPR signals were collected and saved using Biacore T200 Control Software, and data processing was performed using Biacore T200 Evaluation analysis software. The affinity kinetic curves were fitted according to the 1:1 Langmuir binding model, and the KD values were calculated as shown in Table 4 below. The affinities of the chimeric antibodies were superior to that of the control.

**Table 4 Affinity determination of chimeric antibodies**

| Chimeric antibody | KD (M) |
|---|---|
| chi-1 | 5.88×10⁻⁹ |
| chi-2 | 7.27×10⁻¹⁰ |
| chi-3 | 1.87×10⁻⁹ |
| chi-4 | 7.93×10⁻¹⁰ |
| chi-5 | 5.70×10⁻⁹ |
| chi-6 | 1.44×10⁻⁹ |
| chi-7 | 1.59×10⁻⁹ |
| Enfortumab | 6.87×10⁻⁹ |

### Example 7 Determination of Internalization Efficiency of Chimeric Antibodies by Flow Cytometry

MCF-7 cells were collected, and the cell density was adjusted to 2×10⁶ cells/mL with 2% FBS + PBS. 100 µL of the cell suspension was added to each well of a U-bottom 96-well plate. The concentrations of the antibody were adjusted to 40 µg/mL and 8 µg/mL, respectively. 100 µL of each protein solution was added to each well of a U-bottom 96-well plate and mixed evenly, followed by reaction at 4°C for 1 h. After completion of the reaction, the plates were washed two times with PBST, then the cells were resuspended with 200 µL of 2% FBS + PBS and equally divided into two U-bottom 96-well plates. One plate was kept at 4°C, and the other was placed at 37°C, then they were allowed to stand for 20 h. The plates were centrifuged at 2000 r/min for 3 min and washed twice with PBST. Then, 100 µL of APC-conjugated secondary antibody (1:400 dilution) was added to each well and incubated at 4°C for 1 h, followed by washing twice with PBS. The plates were loaded onto the instrument for data analysis. The results are shown in Table 5. Among the seven chimeric antibodies, chi-7 exhibited the highest cellular internalization rate.

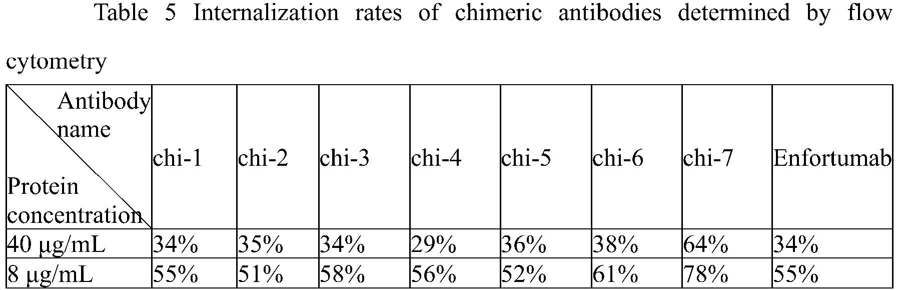

### Example 8 Removal of PTM sites from chimeric antibodies

Since the CDR2 sequence of the chi-7 heavy chain (the amino acid sequence set forth in SEQ ID NO: 16) contains an NG deamidation site and an NTT glycosylation site, which were mutated to SG and NPT, respectively, to remove the deamidation and glycosylation sites (the amino acid sequence set forth in SEQ ID NO: 46), the mutated chi-7 was designated as chi-7-PTM. The heavy chain variable region amino acid sequence is set forth in SEQ ID NO: 47, and the light chain variable region amino acid sequence is set forth in 43. This was linked to the constant region of a human IgG1 antibody and co-transfected with a plasmid in which cVL7 was linked to the human κ light chain constant region to obtain the protein chi-7-PTM. Affinity was determined, and the results are shown in Table 6.

**Table 6 Affinity determination of chi-7-PTM antibody**

| Antibody name | ka(l/Ms) | kd(l/s) | KD (M) |
|---|---|---|---|
| chi-7 | 6.13×10⁵ | 1.08×10⁻³ | 1.76×10⁻⁹ |
| chi-7-PTM | 7.01×10⁵ | 1.47×10⁻³ | 2.09×10⁻⁹ |

### Example 9 Humanization of Chimeric Antibodies

To design humanized antibodies, the chimeric antibody chi-7-PTM (the heavy chain variable region amino acid sequence set forth in SEQ ID NO: 47, and the light chain variable region amino acid sequence set forth in SEQ ID NO: 43) was selected as the template for further humanization design. The humanization of the murine-derived antibody was performed via the complementarity-determining region (CDR) grafting method, as described in U.S. Patent Nos. 4,816,567; 5,225,539; 5,530,101; 5,585,089; 5,693,762; and 6,180,370, and specifically performed according to the following method.

The light chain and heavy chain variable region sequences of antibody chi-7-PTM were aligned against the human immunoglobulin gene database at the NCBI website (http://www.ncbi.nlm.nih.gov/igblast/). Human germline IGVH and IGVK with the highest homology to antibody chi-7-PTM, high expression levels, and prior use in other approved drugs were selected as the frameworks for humanization. The light chain germline acceptor sequence selected for antibody chi-7-PTM was human IGKV7-3*01, and the heavy chain germline acceptor sequence selected was human IGHV1-2*06. Homology modeling was performed on the variable regions of antibody chi-7-PTM, and back mutations were introduced after comparing amino acids within variable region 3Å with the murine antibody. This resulted in five heavy chain variable region sequences, designated VH1, VH2, VH3, VH4, and VH5, and five light chain variable region sequences, designated VL1, VL2, VL3, VL4, and VL5. The sequences are shown in Table 7 below.

**Table 7: Heavy chain and light chain variable region amino acid sequences of chi-7-PTM after back mutations**

| Heavy chain variable region | Amino acid sequence | Light chain variable region | Amino acid sequence |
|---|---|---|---|
| VH1 | SEQ ID NO:48 | VL1 | SEQ ID NO:53 |
| VH2 | SEQ ID NO:49 | VL2 | SEQ ID NO:54 |
| VH3 | SEQ ID NO:50 | VL3 | SEQ ID NO:55 |
| VH4 | SEQ ID NO:51 | VL4 | SEQ ID NO:56 |
| VH5 | SEQ ID NO:52 | VL5 | SEQ ID NO:57 |

### Example 10 Transient Expression of Humanized Antibodies

The humanized heavy chain variable regions were linked to the heavy chain constant region of human IgG1 antibody, and the light chain variable region sequences were linked to the κ light chain constant region (the heavy chain constant region amino acid sequence is set forth in SEQ ID NO: 44, and the light chain constant region amino acid sequence is set forth in SEQ ID NO: 45). A signal peptide sequence and a Kozak sequence were added upstream of the antibody amino acid sequences. DNA sequence optimization and synthesis were performed by GenScript Biotech Co., Ltd. After sequence synthesis, they were cloned into the pCDNA3.4 vector (Invitrogen), and plasmids were extracted. Five humanized heavy chain plasmids and five humanized light chain plasmids were obtained. The heavy chain plasmids and the light chain plasmids were combined in an orthogonal manner and transfected into 293F cells for transient expression, yielding 25 humanized antibodies. The constructed humanized antibodies are shown in Table 8.

**Table 8 Humanized antibodies**

| Humanized antibody | Composition |
|---|---|
| H7-1-1 | VH1+VL1 |
| H7-1-2 | VH1+VL2 |
| H7-1-3 | VH1+VL3 |
| H7-1-4 | VH1+VL4 |
| H7-1-5 | VH1+VL5 |
| H7-2-1 | VH2+VL1 |
| H7-2-2 | VH2+VL2 |
| H7-2-3 | VH2+VL3 |
| H7-2-4 | VH2+VL4 |
| H7-2-5 | VH2+VL5 |
| H7-3-1 | VH3+VL1 |
| H7-3-2 | VH3+VL2 |
| H7-3-3 | VH3+VL3 |
| H7-3-4 | VH3+VL4 |
| H7-3-5 | VH3+VL5 |
| H7-4-1 | VH4+VL1 |
| H7-4-2 | VH4+VL2 |
| H7-4-3 | VH4+VL3 |
| H7-4-4 | VH4+VL4 |
| H7-4-5 | VH4+VL5 |
| H7-5-1 | VH5+VL1 |
| H7-5-2 | VH5+VL2 |
| H7-5-3 | VH5+VL3 |
| H7-5-4 | VH5+VL4 |
| H7-5-5 | VH5+VL5 |

### Example 11 Affinity Determination of Humanized Antibodies

The affinity of antibodies to the antigen Nectin-4-His was determined using Biacore (T200). Ultrapure water was filtered through a 0.22 µm filter membrane, and HBS-EP buffer was prepared. The Nectin-4 humanized antibodies were diluted to 6 µg/mL with HBS-EP buffer as the ligand. Nectin-4-His protein was diluted 2-fold to prepare 8 gradients starting from 100 nM using HBS-EP buffer, and a zero-concentration sample was additionally prepared as the analyte. An appropriate amount of Glycine 1.5 was taken as the regeneration solution. The ligand, analyte, regeneration solution, and HBS-EP buffer were placed on the sample tray. The program was set as follows: ligand flow rate 10 µL/min for 20 s; analyte 30 µL/min, association time 100 s, dissociation time 600 s; regeneration solution 30 µL/min for 30 s. The program was started. SPR signals were collected and saved using Biacore T200 Control Software, and data processing was performed using Biacore T200 Evaluation analysis software. The affinity kinetic curves were fitted according to the 1:1 Langmuir binding model, and the KD values were calculated. The results are shown in Table 9. Based on antibody affinity and the number of amino acid mutations, H7-1-3, H7-1-5, H7-2-3, and H7-2-4 were selected for subsequent experiments.

**Table 9 Affinity determination of humanized antibodies**

| Antibody | KD (M) | kon(l/Ms) | kdis(l/s) |
|---|---|---|---|
| Enfortumab | 9.3117×10⁻⁹ | 9.88×10⁴ | 9.20×10⁻⁴ |
| cHi-7-PTM | 2.9911×10⁻⁹ | 2.483×10⁵ | 7.427×10⁻⁴ |
| H7-1-1 | 7.2382×10⁻⁹ | 4.736×10⁵ | 3.428×10⁻³ |
| H7-1-2 | 7.0759×10⁻⁹ | 4.928×10⁵ | 3.487×10⁻³ |
| H7-1-3 | 3.4106×10⁻⁹ | 2.779×10⁵ | 9.45×10⁻⁴ |
| H7-1-4 | 3.5254×10⁻⁸ | 2.830×10⁵ | 9.977×10⁻³ |
| H7-1-5 | 3.3683×10⁻⁹ | 2.745×10⁵ | 9.246×10⁻⁴ |
| H7-2-1 | 7.2843×10⁻⁹ | 5.505×10⁵ | 4.010×10⁻³ |
| H7-2-2 | 5.3222×10⁻⁹ | 6.146×10⁵ | 3.271×10⁻³ |
| H7-2-3 | 1.4059×10⁻⁹ | 6.942×10⁵ | 0.976×10⁻³ |
| H7-2-4 | 3.8485×10⁻⁹ | 2.918×10⁵ | 1.123×10⁻³ |
| H7-2-5 | 5.3344×10⁻⁹ | 2.542×10⁵ | 1.356×10⁻³ |
| H7-3-1 | 9.1983×10⁻⁹ | 4.827×10⁵ | 4.440×10⁻³ |
| H7-3-2 | 9.1692×10⁻⁹ | 4.851×10⁵ | 4.448×10⁻³ |
| H7-3-3 | 3.8392×10⁻⁹ | 2.873×10⁵ | 1.103×10⁻³ |
| H7-3-4 | 3.9986×10⁻⁹ | 2.821×10⁵ | 1.128×10⁻³ |
| H7-3-5 | 4.0431×10⁻⁹ | 2.644×10⁵ | 1.069×10⁻³ |
| H7-4-1 | 8.3674×10⁻⁹ | 5.084×10⁵ | 4.254×10⁻³ |
| H7-4-2 | 9.5775×10⁻⁹ | 4.971×10⁵ | 4.761×10⁻³ |
| H7-4-3 | 3.5227×10⁻⁹ | 3.151×10⁵ | 1.110×10⁻³ |
| H7-4-4 | 3.4184×10⁻⁹ | 3.136×10⁵ | 1.072×10⁻³ |
| H7-4-5 | 2.8468×10⁻⁸ | 3.297×10⁵ | 9.386×10⁻³ |
| H7-5-1 | 5.3383×10⁻⁹ | 7.139×10⁵ | 3.811×10⁻³ |
| H7-5-2 | 1.3082×10⁻⁸ | 6.146×10⁵ | 8.040×10⁻³ |
| H7-5-3 | 4.7492×10⁻⁹ | 3.649×10⁵ | 1.733×10⁻³ |
| H7-5-4 | 4.7887×10⁻⁹ | 3.738×10⁵ | 1.790×10⁻³ |
| H7-5-5 | 4.7079×10⁻⁹ | 3.543×10⁵ | 1.668×10⁻³ |

The heavy chain and light chain amino acid sequences of H7-1-3 are set forth in SEQ ID NO: 58 and SEQ ID NO: 62, respectively, and the heavy chain and light chain nucleotide sequences of H7-1-3 are set forth in SEQ ID NO: 66 and SEQ ID NO: 70, respectively; the heavy chain and light chain amino acid sequences of H7-1-5 are set forth in SEQ ID NO: 59 and SEQ ID NO: 63, respectively, and the heavy chain and light chain nucleotide sequences of H7-1-5 are set forth in SEQ ID NO: 67 and SEQ ID NO: 71, respectively; the heavy chain and light chain amino acid sequences of H7-2-3 are set forth in SEQ ID NO: 60 and SEQ ID NO: 64, respectively, and the heavy chain and light chain nucleotide sequences of H7-2-3 are set forth in SEQ ID NO: 68 and SEQ ID NO: 72, respectively; the heavy chain and light chain amino acid sequences of H7-2-4 are set forth in SEQ ID NO: 61 and SEQ ID NO: 65, respectively, and the heavy chain and light chain nucleotide sequences of H7-2-4 are set forth in SEQ ID NO: 69 and SEQ ID NO: 73, respectively.

### Example 12 Binding of Humanized Antibodies at the Protein Level

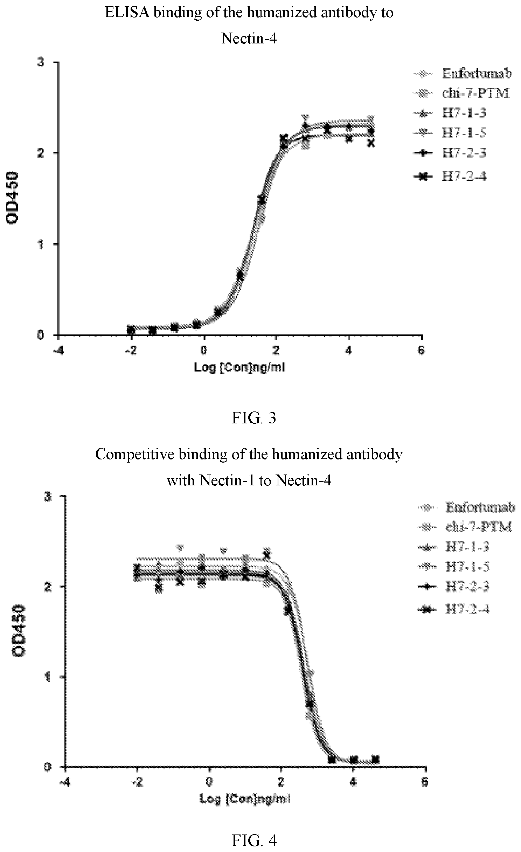

Nectin-4-His protein was diluted to 0.5 µg/mL and coated onto 96-well plates at 100 µL per well, followed by overnight coating at 4°C. The next day, the supernatant was discarded, and 300 µL of 2% non-fat milk was added to block at 37°C for 2 h. After removal of the blocking solution, the plates were washed three times with PBST. The humanized antibodies were diluted to 40 µg/mL, and subjected to 4-fold dilution to prepare 12 gradients, then coated onto 96-well plates at 100 µL per well, followed by reaction at 37°C for 1 h. After completion of the reaction, the plates were washed three times with PBST, and 100 µL of goat anti-human IgG-Fc secondary antibody was added, followed by reaction at 37°C for 1 h. The plates were washed three times with PBST again, then 100 µL of TMB chromogenic substrate solution was added for color development for 10 min. After completion of the reaction, 100 µL of 1 M hydrochloric acid solution was added to each well to terminate the reaction, and the data were read and recorded at 450 nm. The results are shown in FIG. 3 and Table 10. The protein binding ability of the humanized antibodies was not reduced compared to that of the PTM-modified antibody.

**Table 10 Determination of the binding ability of humanized antibodies to Nectin-4**

| Antibody | EC₅₀ value (ng/mL) |
|---|---|
| chi-7-PTM | 22.05 |
| H7-1-3 | 23.18 |
| H7-1-5 | 34.11 |
| H7-2-3 | 20.72 |
| H7-2-4 | 21.43 |
| Enfortumab | 28.45 |

### Example 13 Competitive ELISA for Identifying the Competitive Ability of Humanized Antibodies with Nectin-4 Ligand Nectin1

Nectin-4-His protein was diluted to 2 µg/mL and coated onto 96-well plates at 100 µL per well, followed by overnight coating at 4°C. The next day, the supernatant was discarded, and 300 µL of 2% non-fat milk was added to block at 37°C for 2 h. After removal of the blocking solution, the plates were washed three times with PBST. The humanized antibodies were diluted to 40 µg/mL and subjected to 4-fold dilution to prepare 12 gradients. The diluted antibodies were mixed with the ligand Nectin1 solution (2 µg/mL) at a 1:1 ratio (50 µL each), coated onto 96-well plates, followed by reaction at 37°C for 1 h. After completion of the reaction, the plates were washed three times with PBST, and 100 µL of goat anti-human IgG-Fc secondary antibody was added, followed by reaction at 37°C for 1 h. The plates were washed three times with PBST again, then 100 µL of TMB chromogenic substrate solution was added for color development for 10 min. After completion of the reaction, 100 µL of 1 M hydrochloric acid solution was added to each well to terminate the reaction, and the data were read and recorded at 450 nm. The results are shown in FIG. 4 and Table 11. The results demonstrated that all humanized antibodies were able to compete with Nectin-4 for binding to the ligand Nectin1, with superior efficacy compared to the control.

**Table 11 EC₅₀ Values of humanized antibodies competing with Nectin1 for binding to Nectin-4**

| Antibody | EC₅₀ value (ng/mL) |
|---|---|
| chi-7-PTM | 347.4 |
| H7-1-3 | 429.2 |
| H7-1-5 | 518.6 |
| H7-2-3 | 384.3 |
| H7-2-4 | 385.6 |
| Enfortumab | 545.2 |

### Example 14 Determination of Cell Binding Ability of Humanized Antibodies by Flow Cytometry

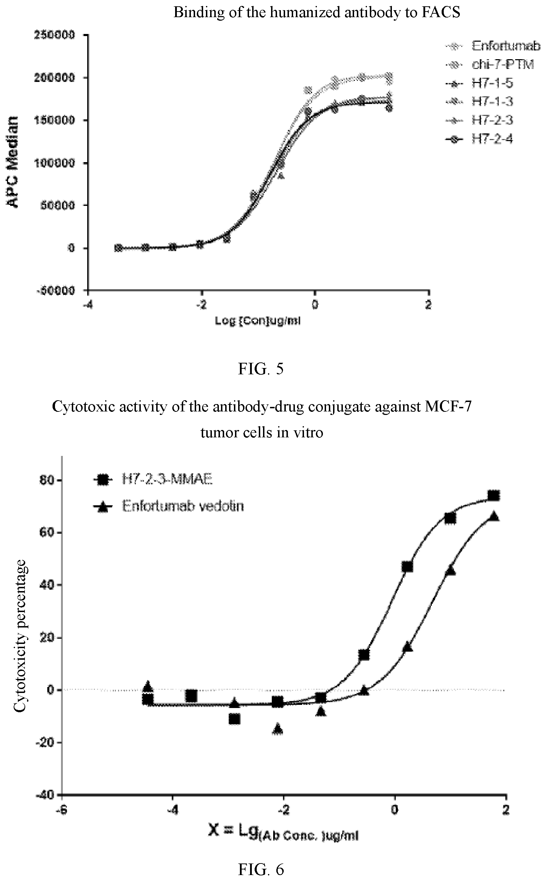

293F-Nectin-4 cells in the logarithmic growth phase were collected and centrifuged at 1200 r/min for 5 min. The supernatant was discarded, and the cells were resuspended in 5 mL of DPBS. The cell density was adjusted to 2×10⁶ cells/mL, and the cells were seeded into U-bottom 96-well plates at 50 µL per well. The antibodies were adjusted to a concentration of 20 µg/mL and serially diluted 3-fold over 12 gradients. The diluted antibodies were added to the U-bottom 96-well plates at 50 µL per well, followed by reaction at 37°C for 1 h. After centrifugation at 1200 r/min for 5 min, the supernatant was discarded, and the plates were washed twice with 200 µL of DPBS. Then, 100 µL of fluorescent secondary antibody in DPBS was added to each well, followed by reaction at 37°C for 1 h. After completion of the reaction, the plates were centrifuged and washed twice with DPBS, and finally 200 µL of DPBS was added to each well to resuspend the cells. The plates were loaded onto the instrument for data analysis. The results are shown in FIG. 5 and Table 12. The EC₅₀ values of H7-1-3, H7-2-3, and H7-2-4 antibodies were all lower than that of the control antibody Enfortumab.

**Table 12 Cell binding ability of humanized antibodies**

| Antibody | EC₅₀ value (µg/mL) |
|---|---|
| chi-7-PTM | 0.1937 |
| H7-1-3 | 0.1568 |
| H7-1-5 | 0.2001 |
| H7-2-3 | 0.1752 |
| H7-2-4 | 0.1580 |
| Enfortumab | 0.1836 |

### Example 15 Determination of Internalization Efficiency of Humanized Antibodies in Tumor Cells by Flow Cytometry

MCF-7 cells were collected, and the cell density was adjusted to 2×10⁶ cells/mL with 2% FBS + PBS. 100 µL of the cell suspension was added to each well of a U-bottom 96-well plate. The humanized antibodies were adjusted to concentrations of 40 µg/mL and 8 µg/mL. 100 µL of each protein solution was added to each well of a U-bottom 96-well plate and mixed evenly, followed by reaction at 4°C for 1 h. After completion of the reaction, the plates were washed two times with PBST, then the cells were resuspended with 200 µL of 2% FBS + PBS and equally divided into two U-bottom 96-well plates. One plate was kept at 4°C, and the other was placed at 37°C, then they were allowed to stand for 20 h. The plates were centrifuged at 2000 r/min for 3 min and washed twice with PBST. Then, 100 µL of APC-conjugated secondary antibody (1:400 dilution) was added to each well and incubated at 4°C for 1 h, followed by washing twice with PBS. Antibody internalization was detected by flow cytometry. The internalization efficiency was calculated as (fluorescence at 4°C-fluorescence at 37°C)/fluorescence at 4°C. The internalization efficiencies of each antibody are shown in Table 13 below. The internalization efficiency of H7-2-3 was 74% at a concentration of 8 µg/mL and 68% at 40 µg/mL, both higher than that of the control antibody Enfortumab.

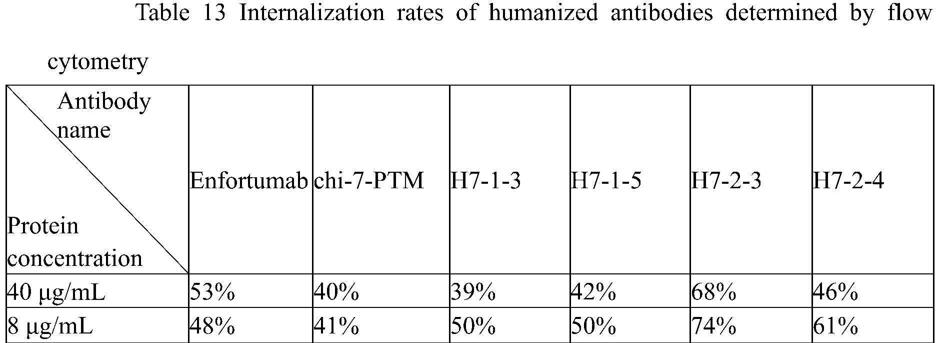

### Example 16 Preparation of Anti-Nectin-4 Antibody-Drug Conjugate

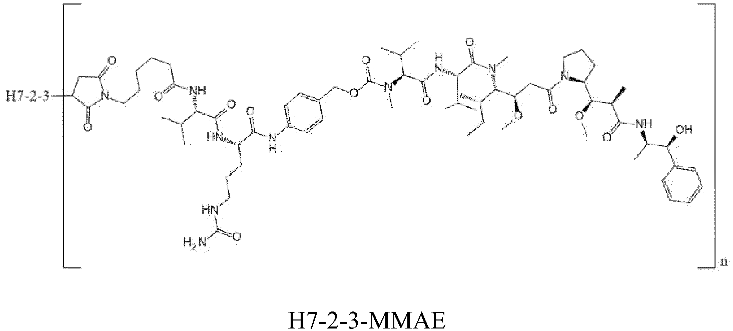

Reduction of the antibody: For the antibody prepared in the examples, antibody H7-2-3 was diluted to a concentration of 18.31 mg/mL. 5.0 mL of this solution was added to a 250 mL glass reaction vessel. Under stirring with a magnetic stirrer, 1.5 µM TCEP aqueous solution (5.83 mL; equivalent to 2.5 equivalents per antibody molecule) was added, and the mixture was stirred at room temperature for 2 h to reduce the disulfide bonds in the hinge region of the antibody.

Conjugation of the antibody with the linker-drug compound: To the above solution system, a DMSO solution (82.88 µL; equivalent to 5.0 equivalents per antibody molecule) containing 3.0 mM of the linker-drug compound (Vc-MMAE) (reference: Lyon RP, Meyer DL, Setter JR, et al. Conjugation of anticancer drugs through endogenous monoclonal antibody cysteine residues. Methods Enzymol. 2012; 502:123-38.doi:10.1016/B978-0-12-416039-2.00006-9) was slowly added. The mixture was stirred at room temperature for 1 h to conjugate the antibody with the linker-drug compound, ultimately yielding the antibody-drug conjugate H7-2-3-MMAE with a DAR value of 4.0.

### Example 17 Cytotoxic Activity of Anti-Nectin-4 Antibody-Drug Conjugate Against Tumor Cells In Vitro

The anti-Nectin-4 antibody-drug conjugate is capable of effectively killing tumor cells expressing the Nectin-4 target. We selected the ATCC-derived breast cancer cell line MCF-7, which expresses Nectin-4, as target cells to evaluate the cytotoxic activity of the anti-Nectin-4 antibody-drug conjugate H7-2-3-MMAE against tumor cells in vitro.

MCF-7 cells were harvested by trypsinization and centrifuged at 1000 rpm for 3 min. The supernatant was discarded, and the cells were resuspended in 10% FBS + EMEM medium. Cell density was adjusted to 1×10⁴ cells/mL, and 100 µL of cell suspension was coated per well in a 96-well plate. Sample preparation: The test sample was diluted with 10% FBS + EMEM medium to 180 µg/mL, followed by 6-fold serial dilution over 9 gradients, with an additional 0 concentration point, totaling 10 concentration points. 50 µL of the mixture was added to each well and mixed with the cells. Each concentration point was set up in triplicate. The plates were incubated at 37°C in a CO₂ incubator for 5 days. After incubation, 50 µL of CTG was added to each well, and chemiluminescence was measured using a multifunctional microplate reader to evaluate cell viability. A four-parameter curve was fitted to calculate the EC₅₀ value for cytotoxicity. The results are shown in FIG. 6 and Table 14. H7-2-3-MMAE exhibited significant cytotoxic activity against tumor cells in vitro, with superior efficacy compared to the control molecule Enfortumab vedotin.

**Table 14 Cytotoxic activity of antibody-drug conjugates**

| Antibody-drug conjugate | EC₅₀ value (µg/mL) |
|---|---|
| H7-2-3-MMAE | 0.8843 |
| Enfortumab vedotin | 4.750 |

### Example 18 In Vivo Efficacy Assay of Anti-Nectin-4 Antibody-Drug Conjugate

### Study on the effect of ADC molecules in a subcutaneous xenograft model of human pancreatic cancer BxPC-3 in female NOG mice

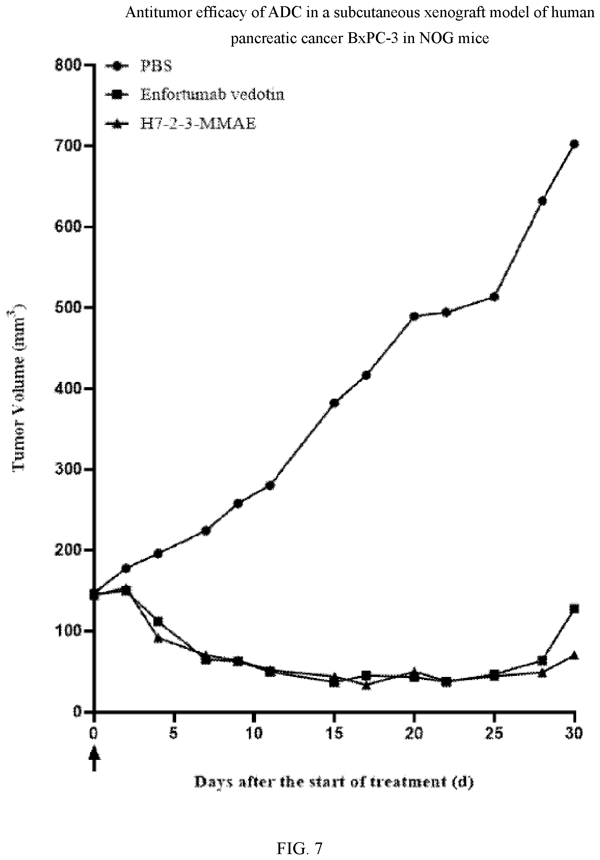

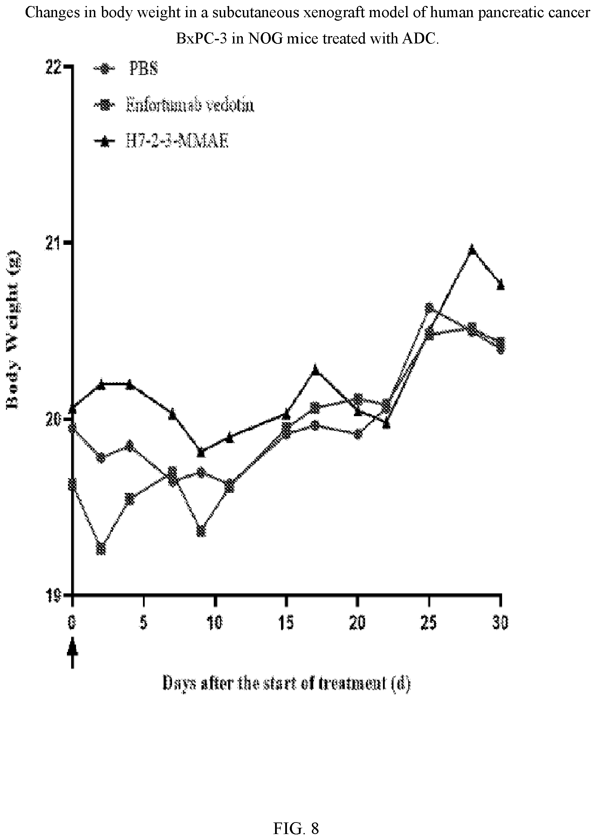

A subcutaneous xenograft model of human pancreatic cancer BxPC-3 was established in female NOG mice to evaluate the therapeutic effect of ADC molecules on the model. Human pancreatic cancer BxPC-3 cells used in this experiment were cultured in RPMI 1640 medium supplemented with 10% FBS in a 37°C incubator with 5% CO₂. Cells were passaged for fewer than ten generations before inoculation into mice. BxPC-3 cells were collected, adjusted to a concentration of 1×10⁸ cells/mL, and mixed with Matrigel at a 1:1 volume ratio. The mixture was inoculated subcutaneously into the right flank of female NOG mice at 0.1 mL per mouse, with an inoculation volume of 5×10⁶ BxPC-3 cells per mouse. When the mean tumor volume reached approximately 145 mm³, mice were randomly divided into 3 groups (6 mice per group) based on tumor volume and body weight: vehicle control PBS group, Enfortumab vedotin group, and H7-2-3-MMAE group. Dosing was initiated on the day of grouping (designated as Day 0), administered via tail vein injection at a dose of 6 mg/kg. A single dose was administered throughout the entire experimental period. Tumor volume and body weight were measured weekly. Results are shown in FIGS. 7 and 8.

Tumor maximum diameter (D) and minimum diameter (d) were measured weekly using an electronic vernier caliper. Tumor volume was calculated, and the tumor growth inhibition rate for each treatment group was calculated according to the formula.$Tumor volume V \left({mm}^{3}\right) = \left[D\times {d}^{2}\right] / 2 ;$

Relative tumor proliferation rate T/C(%)=(Tₙ-T₀)/(Cₙ-C₀)×100%, where Tₙ and Cₙ are the tumor volumes of the treatment group and the control group on Day Dn during the experiment; T₀ and C₀ are the tumor volumes of the treatment group and the control group on Day D0 at the start of the experiment;
$Tumor growth inhibition rate TGI \left(%\right) = 1 - T / C \left(%\right) .$

The results are shown in Table 15. The results demonstrated that the ADC molecules exhibited significant anti-tumor activity, with tumor volume reaching a minimum on Day 17 and the tumor inhibition rate reaching its maximum, after which tumor volume gradually increased. At the same dose, H7-2-3-MMAE demonstrated superior tumor growth inhibition compared to the control molecule Enfortumab vedotin.

**Table 15 Antitumor efficacy of antibody-drug conjugates in vivo**

| Group | Mean tumor volume on D0 (mm³) | Mean tumor volume on D17 (mm³) | Mean tumor volume on D30 (mm³) | TGI on D17 (%) | TGI on D30 (%) |
|---|---|---|---|---|---|
| PBS | 147.3 | 416.8 | 702.5 | / | / |
| Enfortumab vedotin | 145.9 | 55.1 | 127.9 | 133.4% | 103% |
| H7-2-3-MMAE | 144.9 | 33.7 | 70.6 | 141.3% | 113.4% |

## Claims

1. An anti-Nectin-4 antibody or an antigen-binding fragment thereof, wherein the antibody or the antigen-binding fragment thereof comprises a heavy chain variable region and a light chain variable region, the heavy chain variable region comprising heavy chain complementarity-determining regions HCDR1, HCDR2, and HCDR3, and the light chain variable region comprising light chain complementarity-determining regions LCDR1, LCDR2, and LCDR3, wherein: amino acid sequences of HCDR1, HCDR2, and HCDR3 of the heavy chain variable region and amino acid sequences of LCDR1, LCDR2, and LCDR3 of the light chain variable region are selected from the group consisting of:
(1) the amino acid sequences of HCDR1, HCDR2, and HCDR3 are set forth in SEQ ID NO: 1, SEQ ID NO: 2, and SEQ ID NO: 3, respectively; and the amino acid sequences of LCDR1, LCDR2, and LCDR3 are set forth in SEQ ID NO: 18, SEQ ID NO: 19, and SEQ ID NO: 20, respectively;
(2) the amino acid sequences of HCDR1, HCDR2, and HCDR3 are set forth in SEQ ID NO: 4, SEQ ID NO: 5, and SEQ ID NO: 6, respectively; and the amino acid sequences of LCDR1, LCDR2, and LCDR3 are set forth in SEQ ID NO: 21, SEQ ID NO: 22, and SEQ ID NO: 23, respectively;
(3) the amino acid sequences of HCDR1, HCDR2, and HCDR3 are set forth in SEQ ID NO: 7, SEQ ID NO: 8, and SEQ ID NO: 9, respectively; and the amino acid sequences of LCDR1, LCDR2, and LCDR3 are set forth in SEQ ID NO: 24, SEQ ID NO: 19, and SEQ ID NO: 25, respectively;
(4) the amino acid sequences of HCDR1, HCDR2, and HCDR3 are set forth in SEQ ID NO: 7, SEQ ID NO: 10, and SEQ ID NO: 11, respectively; and the amino acid sequences of LCDR1, LCDR2, and LCDR3 are set forth in SEQ ID NO: 26, SEQ ID NO: 27, and SEQ ID NO: 25, respectively;
(5) the amino acid sequences of HCDR1, HCDR2, and HCDR3 are set forth in SEQ ID NO: 7, SEQ ID NO: 12, and SEQ ID NO: 9, respectively; and the amino acid sequences of LCDR1, LCDR2, and LCDR3 are set forth in SEQ ID NO: 24, SEQ ID NO: 19, and SEQ ID NO: 25, respectively;
(6) the amino acid sequences of HCDR1, HCDR2, and HCDR3 are set forth in SEQ ID NO: 13, SEQ ID NO: 14, and SEQ ID NO: 15, respectively; and the amino acid sequences of LCDR1, LCDR2, and LCDR3 are set forth in SEQ ID NO: 28, SEQ ID NO: 29, and SEQ ID NO: 30, respectively;
(7) the amino acid sequences of HCDR1, HCDR2, and HCDR3 are set forth in SEQ ID NO: 13, SEQ ID NO: 16, and SEQ ID NO: 17, respectively; and the amino acid sequences of LCDR1, LCDR2, and LCDR3 are set forth in SEQ ID NO: 28, SEQ ID NO: 29, and SEQ ID NO: 30, respectively;
(8) the amino acid sequences of HCDR1, HCDR2, and HCDR3 are set forth in SEQ ID NO: 13, SEQ ID NO: 46, and SEQ ID NO: 17, respectively; and the amino acid sequences of LCDR1, LCDR2, and LCDR3 are set forth in SEQ ID NO: 28, SEQ ID NO: 29, and SEQ ID NO: 30, respectively.

2. The anti-Nectin-4 antibody or the antigen-binding fragment thereof according to claim 1, wherein an amino acid sequence of the heavy chain variable region comprises an amino acid sequence selected from the group consisting of SEQ ID NOs: 31-37 and SEQ ID NOs: 47-52, and an amino acid sequence of the light chain variable region comprises an amino acid sequence selected from the group consisting of SEQ ID NOs: 38-43 and SEQ ID NOs: 53-57; or an amino acid sequence having at least 80%, 85%, 90%, 95%, 96%, 97%, 98%, or 99% identity to the amino acid sequence selected from the group consisting of SEQ ID NOs: 31-37, SEQ ID NOs: 47-52, SEQ ID NOs: 38-43, and SEQ ID NOs: 53-57.

3. The anti-Nectin-4 antibody or the antigen-binding fragment thereof according to claim 2, wherein the amino acid sequence of the heavy chain variable region and the amino acid sequence of the light chain variable region of the antibody are selected from the group consisting of:
(1) the amino acid sequence of the heavy chain variable region set forth in SEQ ID NO: 31, and the amino acid sequence of the light chain variable region set forth in SEQ ID NO: 38; or an amino acid sequence having at least 80%, 85%, 90%, 95%, 96%, 97%, 98%, or 99% identity to the amino acid sequences;
(2) the amino acid sequence of the heavy chain variable region set forth in SEQ ID NO: 32, and the amino acid sequence of the light chain variable region set forth in SEQ ID NO: 39; or an amino acid sequence having at least 80%, 85%, 90%, 95%, 96%, 97%, 98%, or 99% identity to the amino acid sequences;
(3) the amino acid sequence of the heavy chain variable region set forth in SEQ ID NO: 33, and the amino acid sequence of the light chain variable region set forth in SEQ ID NO: 40; or an amino acid sequence having at least 80%, 85%, 90%, 95%, 96%, 97%, 98%, or 99% identity to the amino acid sequences;
(4) the amino acid sequence of the heavy chain variable region set forth in SEQ ID NO: 34, and the amino acid sequence of the light chain variable region set forth in SEQ ID NO: 41; or an amino acid sequence having at least 80%, 85%, 90%, 95%, 96%, 97%, 98%, or 99% identity to the amino acid sequences;
(5) the amino acid sequence of the heavy chain variable region set forth in SEQ ID NO: 35, and the amino acid sequence of the light chain variable region set forth in SEQ ID NO: 40; or an amino acid sequence having at least 80%, 85%, 90%, 95%, 96%, 97%, 98%, or 99% identity to the amino acid sequences;
(6) the amino acid sequence of the heavy chain variable region set forth in SEQ ID NO: 36, and the amino acid sequence of the light chain variable region set forth in SEQ ID NO: 42; or an amino acid sequence having at least 80%, 85%, 90%, 95%, 96%, 97%, 98%, or 99% identity to the amino acid sequences;
(7) the amino acid sequence of the heavy chain variable region set forth in SEQ ID NO: 37, and the amino acid sequence of the light chain variable region set forth in SEQ ID NO: 43; or an amino acid sequence having at least 80%, 85%, 90%, 95%, 96%, 97%, 98%, or 99% identity to the amino acid sequences;
(8) the amino acid sequence of the heavy chain variable region set forth in SEQ ID NO: 47, and the amino acid sequence of the light chain variable region set forth in SEQ ID NO: 43; or an amino acid sequence having at least 80%, 85%, 90%, 95%, 96%, 97%, 98%, or 99% identity to the amino acid sequences;
(9) the amino acid sequence of the heavy chain variable region set forth in SEQ ID NO: 48, and the amino acid sequence of the light chain variable region set forth in SEQ ID NO: 53; or an amino acid sequence having at least 80%, 85%, 90%, 95%, 96%, 97%, 98%, or 99% identity to the amino acid sequences;
(10) the amino acid sequence of the heavy chain variable region set forth in SEQ ID NO: 48, and the amino acid sequence of the light chain variable region set forth in SEQ ID NO: 54; or an amino acid sequence having at least 80%, 85%, 90%, 95%, 96%, 97%, 98%, or 99% identity to the amino acid sequences;
(11) the amino acid sequence of the heavy chain variable region set forth in SEQ ID NO: 48, and the amino acid sequence of the light chain variable region set forth in SEQ ID NO: 55; or an amino acid sequence having at least 80%, 85%, 90%, 95%, 96%, 97%, 98%, or 99% identity to the amino acid sequences;
(12) the amino acid sequence of the heavy chain variable region set forth in SEQ ID NO: 48, and the amino acid sequence of the light chain variable region set forth in SEQ ID NO: 56; or an amino acid sequence having at least 80%, 85%, 90%, 95%, 96%, 97%, 98%, or 99% identity to the amino acid sequences;
(13) the amino acid sequence of the heavy chain variable region set forth in SEQ ID NO: 48, and the amino acid sequence of the light chain variable region set forth in SEQ ID NO: 57; or an amino acid sequence having at least 80%, 85%, 90%, 95%, 96%, 97%, 98%, or 99% identity to the amino acid sequences;
(14) the amino acid sequence of the heavy chain variable region set forth in SEQ ID NO: 49, and the amino acid sequence of the light chain variable region set forth in SEQ ID NO: 53; or an amino acid sequence having at least 80%, 85%, 90%, 95%, 96%, 97%, 98%, or 99% identity to the amino acid sequences;
(15) the amino acid sequence of the heavy chain variable region set forth in SEQ ID NO: 49, and the amino acid sequence of the light chain variable region set forth in SEQ ID NO: 54; or an amino acid sequence having at least 80%, 85%, 90%, 95%, 96%, 97%, 98%, or 99% identity to the amino acid sequences;
(16) the amino acid sequence of the heavy chain variable region set forth in SEQ ID NO: 49, and the amino acid sequence of the light chain variable region set forth in SEQ ID NO: 55; or an amino acid sequence having at least 80%, 85%, 90%, 95%, 96%, 97%, 98%, or 99% identity to the amino acid sequences;
(17) the amino acid sequence of the heavy chain variable region set forth in SEQ ID NO: 49, and the amino acid sequence of the light chain variable region set forth in SEQ ID NO: 56; or an amino acid sequence having at least 80%, 85%, 90%, 95%, 96%, 97%, 98%, or 99% identity to the amino acid sequences;
(18) the amino acid sequence of the heavy chain variable region set forth in SEQ ID NO: 49, and the amino acid sequence of the light chain variable region set forth in SEQ ID NO: 57; or an amino acid sequence having at least 80%, 85%, 90%, 95%, 96%, 97%, 98%, or 99% identity to the amino acid sequences;
(19) the amino acid sequence of the heavy chain variable region set forth in SEQ ID NO: 50, and the amino acid sequence of the light chain variable region set forth in SEQ ID NO: 53; or an amino acid sequence having at least 80%, 85%, 90%, 95%, 96%, 97%, 98%, or 99% identity to the amino acid sequences;
(20) the amino acid sequence of the heavy chain variable region set forth in SEQ ID NO: 50, and the amino acid sequence of the light chain variable region set forth in SEQ ID NO: 54; or an amino acid sequence having at least 80%, 85%, 90%, 95%, 96%, 97%, 98%, or 99% identity to the amino acid sequences;
(21) the amino acid sequence of the heavy chain variable region set forth in SEQ ID NO: 50, and the amino acid sequence of the light chain variable region set forth in SEQ ID NO: 55; or an amino acid sequence having at least 80%, 85%, 90%, 95%, 96%, 97%, 98%, or 99% identity to the amino acid sequences;
(22) the amino acid sequence of the heavy chain variable region set forth in SEQ ID NO: 50, and the amino acid sequence of the light chain variable region set forth in SEQ ID NO: 56; or an amino acid sequence having at least 80%, 85%, 90%, 95%, 96%, 97%, 98%, or 99% identity to the amino acid sequences;
(23) the amino acid sequence of the heavy chain variable region set forth in SEQ ID NO: 50, and the amino acid sequence of the light chain variable region set forth in SEQ ID NO: 57; or an amino acid sequence having at least 80%, 85%, 90%, 95%, 96%, 97%, 98%, or 99% identity to the amino acid sequences;
(24) the amino acid sequence of the heavy chain variable region set forth in SEQ ID NO: 51, and the amino acid sequence of the light chain variable region set forth in SEQ ID NO: 53; or an amino acid sequence having at least 80%, 85%, 90%, 95%, 96%, 97%, 98%, or 99% identity to the amino acid sequences;
(25) the amino acid sequence of the heavy chain variable region set forth in SEQ ID NO: 51, and the amino acid sequence of the light chain variable region set forth in SEQ ID NO: 54; or an amino acid sequence having at least 80%, 85%, 90%, 95%, 96%, 97%, 98%, or 99% identity to the amino acid sequences;
(26) the amino acid sequence of the heavy chain variable region set forth in SEQ ID NO: 51, and the amino acid sequence of the light chain variable region set forth in SEQ ID NO: 55; or an amino acid sequence having at least 80%, 85%, 90%, 95%, 96%, 97%, 98%, or 99% identity to the amino acid sequences;
(27) the amino acid sequence of the heavy chain variable region set forth in SEQ ID NO: 51, and the amino acid sequence of the light chain variable region set forth in SEQ ID NO: 56; or an amino acid sequence having at least 80%, 85%, 90%, 95%, 96%, 97%, 98%, or 99% identity to the amino acid sequences;
(28) the amino acid sequence of the heavy chain variable region set forth in SEQ ID NO: 51, and the amino acid sequence of the light chain variable region set forth in SEQ ID NO: 57; or an amino acid sequence having at least 80%, 85%, 90%, 95%, 96%, 97%, 98%, or 99% identity to the amino acid sequences;
(29) the amino acid sequence of the heavy chain variable region set forth in SEQ ID NO: 52, and the amino acid sequence of the light chain variable region set forth in SEQ ID NO: 53; or an amino acid sequence having at least 80%, 85%, 90%, 95%, 96%, 97%, 98%, or 99% identity to the amino acid sequences;
(30) the amino acid sequence of the heavy chain variable region set forth in SEQ ID NO: 52, and the amino acid sequence of the light chain variable region set forth in SEQ ID NO: 54; or an amino acid sequence having at least 80%, 85%, 90%, 95%, 96%, 97%, 98%, or 99% identity to the amino acid sequences;
(31) the amino acid sequence of the heavy chain variable region set forth in SEQ ID NO: 52, and the amino acid sequence of the light chain variable region set forth in SEQ ID NO: 55; or an amino acid sequence having at least 80%, 85%, 90%, 95%, 96%, 97%, 98%, or 99% identity to the amino acid sequences;
(32) the amino acid sequence of the heavy chain variable region set forth in SEQ ID NO: 52, and the amino acid sequence of the light chain variable region set forth in SEQ ID NO: 56; or an amino acid sequence having at least 80%, 85%, 90%, 95%, 96%, 97%, 98%, or 99% identity to the amino acid sequences;
(33) the amino acid sequence of the heavy chain variable region set forth in SEQ ID NO: 52, and the amino acid sequence of the light chain variable region set forth in SEQ ID NO: 57; or an amino acid sequence having at least 80%, 85%, 90%, 95%, 96%, 97%, 98%, or 99% identity to the amino acid sequences.

4. The anti-Nectin-4 antibody or the antigen-binding fragment thereof according to claim 3, wherein the amino acid sequences of the heavy chain variable region and the light chain variable region of the antibody are selected from the group consisting of:
(1) the amino acid sequence of the heavy chain variable region set forth in SEQ ID NO: 37, and the amino acid sequence of the light chain variable region set forth in SEQ ID NO: 43;
(2) the amino acid sequence of the heavy chain variable region set forth in SEQ ID NO: 47, and the amino acid sequence of the light chain variable region set forth in SEQ ID NO: 43;
(3) the amino acid sequence of the heavy chain variable region set forth in SEQ ID NO: 48, and the amino acid sequence of the light chain variable region set forth in SEQ ID NO: 55;
(4) the amino acid sequence of the heavy chain variable region set forth in SEQ ID NO: 48, and the amino acid sequence of the light chain variable region set forth in SEQ ID NO: 57;
(5) the amino acid sequence of the heavy chain variable region set forth in SEQ ID NO: 49, and the amino acid sequence of the light chain variable region set forth in SEQ ID NO: 55;
(6) the amino acid sequence of the heavy chain variable region set forth in SEQ ID NO: 49, and the amino acid sequence of the light chain variable region set forth in SEQ ID NO: 56.

5. The anti-Nectin-4 antibody or the antigen-binding fragment thereof according to any one of claims 1 to 4, wherein the antibody or the antigen-binding fragment thereof further comprises a complete structure having two light chains and two heavy chains, and the antibody is selected from the group consisting of:
(1) a heavy chain amino acid sequence set forth in SEQ ID NO: 58, and a light chain amino acid sequence set forth in SEQ ID NO: 62;
(2) a heavy chain amino acid sequence set forth in SEQ ID NO: 59, and a light chain amino acid sequence set forth in SEQ ID NO: 63;
(3) a heavy chain amino acid sequence set forth in SEQ ID NO: 60, and a light chain amino acid sequence set forth in SEQ ID NO: 64;
(4) a heavy chain amino acid sequence set forth in SEQ ID NO: 61, and a light chain amino acid sequence set forth in SEQ ID NO: 65.

6. A polynucleotide, wherein the polynucleotide encodes the anti-Nectin-4 antibody or the antigen-binding fragment thereof according to any one of claims 1 to 5.

7. An expression vector, comprising the polynucleotide according to claim 6.

8. An antibody conjugate, wherein the antibody conjugate has a structure represented by the following formula: 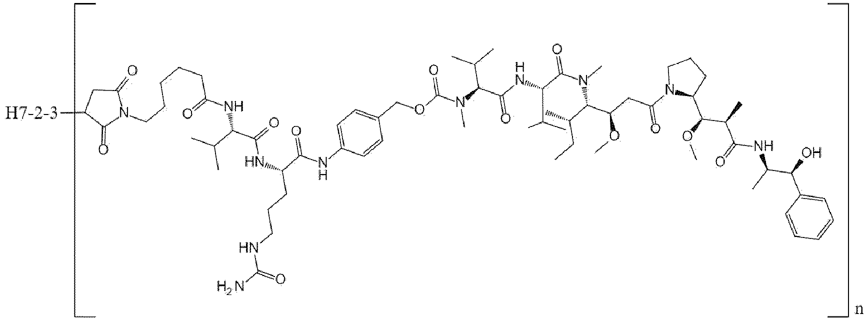 wherein n is 1 to 8;
H7-2-3 is an anti-Nectin-4 antibody or an antigen-binding fragment thereof, and the anti-Nectin-4 antibody comprises a heavy chain variable region and a light chain variable region, wherein amino acid sequences of HCDR1, HCDR2, and HCDR3 of the heavy chain variable region are set forth in SEQ ID NO: 13, SEQ ID NO: 46, and SEQ ID NO: 17, respectively, and amino acid sequences of LCDR1, LCDR2, and LCDR3 of the light chain variable region are set forth in SEQ ID NO: 28, SEQ ID NO: 29, and SEQ ID NO: 30, respectively.

9. The antibody conjugate according to claim 8, wherein the antibody has a heavy chain variable region sequence set forth in SEQ ID NO: 49 and a light chain variable region sequence set forth in SEQ ID NO: 55.

10. A pharmaceutical composition, wherein the pharmaceutical composition comprises the anti-Nectin-4 antibody or the antigen-binding fragment thereof according to any one of claims 1 to 5 and/or the antibody conjugate according to any one of claims 8 to 9, and a pharmaceutically acceptable carrier.

11. Use of the anti-Nectin-4 antibody or the antigen-binding fragment thereof according to any one of claims 1 to 5, the antibody conjugate according to any one of claims 8 to 9, the polynucleotide according to claim 6, the expression vector according to claim 7, or the pharmaceutical composition according to claim 10, in the manufacture of a medicament for treatment or prevention of cancer.

12. The cancer according to claim 11, wherein the cancer is selected from bladder cancer, breast cancer, ovarian cancer, pancreatic cancer, hepatocellular carcinoma, gastric cancer, non-Hodgkin's lymphoma, Hodgkin's lymphoma, acute lymphoblastic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, a gastrointestinal tumor, prostate cancer, colorectal cancer, glioma, and mesothelioma.
